# EUROPÄISCHE PATENTSCHRIFT

(11) **EP 0 377 015 B1**
(45) Veröffentlichungstag und Bekanntmachung des Hinweises auf die Patenterteilung: **30.11.1994**
(21) Anmeldenummer: 89906358.0
(22) Anmeldetag: 24.05.1989
(51) Int. Cl.: C12Q 1/68, C12N 15/09

(54) **OLIGONUCLEOTIDBANK UND VERFAHREN ZUR DNA-SEQUENZIERUNG**
OLIGONUCLEOTIDE BANK AND PROCESS FOR DNA SEQUENCING
BANQUE D'OLIGONUCLEOTIDES ET PROCEDE DE SEQUENCAGE D'ADN

(30) Priorität: 24.05.1988 DE 3817591
(43) Veröffentlichungstag der Anmeldung: 11.07.1990
(73) Patentinhaber: Gesellschaft für Biotechnologische Forschung mbH (GBF), D-38124 Braunschweig (DE)
(72) Erfinder: BLÖCKER, Helmut, D-3300 Braunschweig (DE)
(74) Vertreter: Boeters, Hans Dietrich, Dr.
(86) Internationale Anmeldenummer: EP8900579
(87) Internationale Veröffentlichungsnummer: WO8911211

(56) Entgegenhaltungen:
- EP-A- 0 114 599
- DE-A- 3 312 929
- GENE, Band 90, 1990; Seiten 177-178#
- PNAS, USA, Band 86, September 1989; Seiten 6917-6921#
- RAHM, "Pharmacia Molecular Biological Catalogue", May 1986, Lund (SE); Seiten 95-107#
- DNA, Band 3, Nr. 4, 1984, Mary Ann Liebert Inc., New York, NY (US); R. SANCHEZ-PESCADOR et al., Seiten 339-343#

## Beschreibung

Die Sequenzanalyse von Nucleinsäurefragmenten, insbesondere von DNA, ist als ein Schlüsselverfahren der modernen Biowissenschaften und der modernen Biotechnologie anzusehen. In letzter Zeit gerät mit der ständigen Verfeinerung der Sequenzierungstechniken auch die Analyse von ganzen, komplexen Genomen in den Bereich des Möglichen.

Neben einigen weniger populären Methoden, wie der massen spektrometrischen Sequenzierung nach der DE-A-3 312 929; werden heutzutage vor allem zwei grundsätzlich unterschiedliche Methoden zur Sequenzanalyse angewandt, und zwar
- die Sequenzierung durch chemische Modifizierung und Abbau (Maxam/Gilbert-Methode) sowie
- die Sequenzierung durch kontrollierten Polymerase-Einbau unterschiedlicher Nucleotide (Sanger-Methode); vgl. beispielsweise Gassen & Schäfer, Sequenzbestimmung von Nucleinsäuren und Proteinen, in: Gassen et al, Gentechnik, 2. Auflage, Gustav-Fischer-Verlag, 1987, Seite 241 ff.

Die Sanger-Methode kann sowohl mit einzelsträngiger DNA, die speziell für den Zweck der Sequenzierung hergestellt wird, als auch seit wenigen Jahren mit jeder ausreichend reinen doppelsträngigen DNA durchgeführt werden. Ein durchgehendes Wesensmerkmal der Sanger-Methode bleibt jedoch unter anderem die Abhängigkeit der verwendeten Einbauenzyme (DNA-Polymerasen) sowohl von einer Matrize (Templat = zu sequenzierende DNA) als auch von einem Startmolekül (Primer). Der Primer ist in der Regel ein kurzes, chemisch synthetisiertes Oligonucleotid, das in seiner ganzen Länge zu einem 3' von dem zu sequenzierenden DNA-Abschnitt gelegenen Teilstück basenkomplementär ist: 
Primer
5′ zu sequenzieren
3′
Die Anheftungsstelle (Hybridisierungsstelle) des Primers muß derart gewählt sein, daß unter den experimentellen Bedingungen der Primer nur die gewünschte und keine einzige weitere Anheftungsstelle findet, um ein lesbares Sequenzierungsergebnis erreichen zu können. Die Spezifität des Primers hängt direkt von seiner Länge und der Komplexität der im Experiment verwendeten DNA ab. Aus statistischen Erwägungen und praktischer Erfahrung ergibt sich, daß Primer mit einer Kettenlänge von bis zu 24 Basen für alle denkbaren Fälle ausreichen sollten.

Bei der Sequenzanalyse sehr langer DNA (beispielsweise bei Genomsequenzierungen) werden gegenwärtig im wesentlichen zwei verschiedene Strategien verfolgt.

Strategie 1: Die Ein-Primer-Methode. Hierbei wird die zu sequenzierende DNA (je nach Verfahrensvariante) mehr oder weniger in Zufallsfragmente oder teilweise geordnete Fragmente zerlegt, wonach die Fragmente in ein und denselben Vektor inseriert werden. Nach Transformation von Zellen werden DNA-Präparationen von einzelnen Klonen hergestellt und der Sequenzanalyse unterworfen. Da sich alle DNA-Präparationen maximal um die inserierte DNA unterscheiden, kann für alle anstehenden Sequenzanalysen im Prinzip ein einziger Primer verwendet werden, der beispielsweise direkt neben der Insertionsstelle auf der Vektor-DNA hybridisiert. Diesem Vorteil stehen jedoch auch erhebliche Nachteile gegenüber,
- denn da die Klone zufällig ausgesucht werden müssen, werden einzelne Abschnitte der Original-DNA sehr oft weit häufiger als nötig sequenziert, und
- schließlich klaffen in der mit Hilfe von geeigneten Computer-Programmen zu einer Gesamtsequenz geordneten Sammlung der Teilsequenzierungsergebnisse häufig beträchtliche Lücken, da beispielsweise gewisse Abschnitte der zu analysierenden Original-DNA "schwer klonierbar" sind.

Strategie 2: Die Mehr-Primer-Methode. Die zu sequenzierende DNA wird hier nicht wie bei Strategie 1 nach der Schrotschuß-Methode, sondern gezielt progressierend; vgl, z.B. DNA, 3 (1984) 339. Der in einem Experiment noch sicher zu entschlüsselnde Sequenzabschnitt dient zur Auswahl einer Primer-Anheftungsstelle für das nächste Experiment und so fort. Der Vorteil dieser Methode liegt vor allem darin, daß unnötige Mehrfachsequenzanalysen wie bei der Strategie 1 vermieden werden. Der wohl wesentliche Grund, warum diese Methode nicht allein für die Analyse langer DNA-Abschnitte verwendet wird, liegt in der Beschränkung durch die gegenwärtig verfügbaren Methoden zur chemischen DNA-Synthese.

Es hat nicht an Versuchen gefehlt, die jeweiligen Nachteile der oben beschriebenen Verfahren zu mildern, beispielsweise durch das "Multiplex-Sequencing" bei Strategie 1 und das simultane progressierende Sequenzieren von verschiedenen Stellen aus bei Strategie 2. Ein Weg, den wesentlichen Nachteil der Strategie 2 ganz zu beseitigen, liegt theoretisch darin, eine Bank aller denkbaren Primer anzulegen. Dies ist jedoch durch direkte chemische Synthese praktisch nicht möglich, da es allein 4²⁴ verschiedene Primer der Kettenlänge 24 gibt.

Erfindungsgemäß wird daher vorgeschlagen, kürzere, chemisch synthetisierte Oligonucleotide zu verwenden. Da jede längere Sequenz im Prinzip aus zwei oder mehr kürzeren Sequenzen zusammensetzbar ist, werden die dem gewünschten Primer entsprechenden kurzen Oligonucleotide anstelle des gewünschten Primers zu der zu sequenzierenden DNA gegeben und durch eine
geeignete Prozedur, beispielsweise mit T4-DNA-Ligase, zu dem gewünschten Primer zusammengefügt. Die Sequenzierungsreaktion kann dann anschließend praktisch unter Standardbedingungen durchgeführt werden.

Die der Erfindung zugrundeliegende Aufgabe wird durch die Gegenstände der Ansprüche gelöst.

### Beispiel 1

In typischen Experimenten wurden ca. 2,5 pMol DNA des Plasmids pTZ18R (Pharmacia-LKB) mit NaOH denaturiert, mit Ethanol gefällt, getrocknet und in Wasser aufgenommen. Die DNA-Lösung wurde mit je 2,5 pMol zweier verschiedener Oligonucleotidlösungen versetzt. Die Oligonucleotide der Kettenlänge 8 (Octamere) waren derart gewählt, daß sie unmittelbar benachbart auf einem Abschnitt bekannter Nucleotidsequenz der Plasmid-DNA hybridisieren konnten. Das 5'-Ende des im hybridisierten Zustand benachbart zum 3'-Ende des anderen Oligonucleotids gelegenen Octamers war zuvor nach gängigen Verfahren mit T4-Polynucleotid-Kinase und ATP phosphoryliert worden. Nach Einstellen der bekannten Pufferbedingungen für Ligationen mit T4-DNA-Ligase und Versetzen mit 1 Einheit des Enzyms wurde die Lösung (10 µl Endvolumen) 4 h lang bei 15 °C inkubiert. Diese Ligationslösung wurde vor der Sequenzierung 5 min lang bei 37 °C aufbewahrt, um möglichst viele durch Ligation entstandene 16-mere und möglichst wenige 8-mere hybridisieren zu lassen.

Die folgende Sequenzierung sollte dazu dienen, die Sequenz des gewählten Vektors zu überprüfen.

Dabei wurde nach folgenden Anweisungen des Standardprotokolls der Firma United States Biochemical Corporation (USB) gearbeitet, wobei 8 µl der Ligationslösung eingesetzt wurden:

### Markierungsreaktion

Zum verschweißten Template-Primer gab man die folgenden Komponenten (auf Eis):

| | |
|---|---|
| Template-Primer | 10,0 µl |
| DTT 0,1 M | 1,0 µl |
| Verdünnte Markierungsmischung | 2,0 µl |
| [α-³⁵ S] d ATP | 0,5 µl |
| Verdünnte Sequenase Version 2,0 (das Enzym ist stets zuletzt zuzugeben) | 2,0 µl |

Man mischte sorgfältig (wobei man Blasen vermied) und inkubierte 2 bis 5 min lang bei Raumtemperatur (gegebenenfalls auch darunter; eine zu lange oder zu warme Inkubierung führt zu Sequenzierungsartefakten in 100 Basen des Primers). Man konnte entweder [α-¹² P] d ATP oder [α-³⁵ S] d ATP verwenden. Nominal setzte man 0,5 µl 10 µCi/µl und 10 µM (1000 Ci/mmol) d ATP ein.

### Terminierungsreaktion

1. Man bezeichnete 4 Röhrchen mit G, A, T und C.
2. Man gab 2,5 µl der dd GTP-Terminationsmischung in das mit G bezeichnete Röhrchen. Gleichzeitig füllte man die mit A, T und C bezeichneten Röhrchen mit 2,5 µl dd ATP, dd TTP bzw. dd CTP Terminationsmischungen.
3. Man wärmte die Röhrchen auf 37 °C mindestens eine Minute vor.
4. Wenn die Markierungsinkubation vollständig war, entfernte man 3,5 µl und überführte sie in das mit G bezeichnete Röhrchen. Man mischte, zentrifugierte und führte die Inkubierung bei dem mit G bezeichnete Röhrchen bei 37 °C fort. In entsprechender Weise überführte man 3,5 µl der Markierungsreaktion in die A, T und C bezeichnete Röhrchen, mischte und führte sie in das 37 °C warme Bad zurück.
5. Man führte die Inkubationen insgesamt 3 bis 5 min fort.
6. Man gab 4 µl einer Stoplösung zu jeder Terminierungsreaktion, mischte sorgfältig und lagerte sie auf Eis, bis das Sequenzierungsgel beschickt werden konnte.
7. Sobald das Gel zum Beschicken bereit war, erwärmte man die Proben auf 75 bis 80 °C 2 min lang und beschickte sofort das Gel. Man verwendete 2 bis 3 µl für jede Bahn.

Abweichend von diesem Protokoll wurde auf das "Annealing" von Templat und Primer verzichtet.

Sofern die genannte Phosphorylierung in Gegenwart von [gamma-³²P]ATP durchgeführt worden war, wurde auf die spätere Verwendung von [α-³²P]dATP verzichtet.

## Patentansprüche

1. Verfahren zur DNA-Sequenzierung, nach der Mehr-Primer-Methode, dadurch ***gekennzeichnet***, daß man
(a) bei der zu sequenzierenden DNA eine einzelsträndige Primeranheftungsstelle bekannter Sequenz wählt,
(b) auf dieser Primeranheftungsstelle unmittelbar benachbart zwei kurze Oligonucleotide, bei denen es sich um hexamere, heptamere, octamere oder nonamere Oligonucleotide handelt, hybridisiert und
(c) ggf. gleichzeitig mit Stufe (b) oder nach Stufe (b) ein weiteres hexameres, heptameres, octameres oder nonameres Oligonucleotid unmittelbar benachbart zu einem der beiden anderen Oligonucleotide auf der Primeranheftungsstelle hybridisiert und
(d) die Oligonucleotide zu einem Primer ligiert.

2. Verfahren nach Anspruch 1, dadurch ***gekennzeichnet***, daß man auf der Primeranheftungsstelle zwei Oligonucleotide hybridisiert und miteinander zu einem Prä-Primer ligiert und erst danach auf der Primeranheftungsstelle ein drittes Oligomeres hybridisiert und mit dem Prä-Primer ligiert.

3. Verfahren nach Anspruch 1 oder 2, dadurch ***gekennzeichnet***, daß man für die Primerbildung hexamere, heptamere, octamere und/oder nonamere Oligonucleotide verwendet.

4. Verfahren nach einem der vorhergehenden Ansprüche, dadurch ***gekennzeichnet***, daß man Oligonucleotide verwendet, die an ihrem 5'-Ende, sofern dieses Ende mit einem benachbarten Oligonucleotid mit Hilfe von T4-DNA-Ligase ligiert werden soll, phosphoryliert sind.

5. Verfahren nach einem der vorhergehenden Ansprüche, dadurch ***gekennzeichnet***, daß man nach der Ligation und vor der Sequenzierung nicht zum gewünschten Primer ligierte Oligonucleotide durch Wärmebehandlung entfernt.

6. Oligonucleotidbank, zur Durchführung des Verfahrens gemäß einem der vorhergehenden Ansprüche, umfassend alle denkbaren 4⁶ verschiedenen hexameren Oligonucleotide.

7. Oligonucleotidbank, zur Durchführung des Verfahrens gemäß einem der vorhergehenden Ansprüche, umfassend alle denkbaren 4⁷ verschiedenen heptameren Oligonucleotide.

8. Oligonucleotidbank, zur Durchführung des Verfahrens gemäß einem der vorhergehenden Ansprüche, umfassend alle denkbaren 4⁸ verschiedenen octameren Oligonucleotide.

9. Oligonucleotidbank, zur Durchführung des Verfahrens gemäß einem der vorhergehenden Ansprüche, umfassend alle denkbaren 4⁹ verschiedenen nonameren Oligonucleotide.

## Claims

1. Process for DNA sequencing by the multi-primer method, characterised in that
(a) in the DNA to be sequenced a single-stranded primer attachment site of known sequence is selected;
(b) two short oligonucleotides, which may be hexameric, heptameric, octameric or nonameric oligonucleotides, are hybridised immediately adjacent to one another at that primer attachment site, and
(c) if appropriate, simultaneously with step (b) or after step (b) a further hexameric, heptameric, octameric or nonameric oligonucleotide is hybridised immediately adjacent to one of the other two oligonucleotides at the primer attachment site, and
(d) the oligonucleotides are ligated to form a primer.

2. Process according to claim 1, characterised in that at the primer attachment site two oligonucleotides are hybridised and ligated with one another to form a pre-primer and only then is a third oligomer hybridised and ligated to the pre-primer at the primer attachment site.

3. Process according to claim 1 or claim 2, characterised in that hexameric, heptameric, octameric and/or nonameric oligonucleotides are used for the primer formation.

4. Process according to any one of the preceding claims, characterised in that there are used oligonucleotides that are phosphorylated at their 5' end when that end is to be ligated with an adjacent oligonucleotide with the aid of T4-DNA ligase.

5. Process according to any one of the preceding claims, characterised in that after the ligation and before the sequencing oligonucleotides that have not be ligated to form the desired primer are removed by heat treatment.

6. Oligonucleotide bank, for carrying out the process according to any one of the preceding claims, comprising all possible 4⁶ different hexameric oligonucleotides.

7. Oligonucleotide bank, for carrying out the process according to any one of the preceding claims, comprising all possible 4⁷ different heptameric oligonucleotides.

8. Oligonucleotide bank, for carrying out the process according to any one of the preceding claims, comprising all possible 4⁸ different octameric oligonucleotides.

9. Oligonucleotide bank, for carrying out the process according to any one of the preceding claims, comprising all possible 4⁹ different nonameric oligonucleotides.

## Revendications

1. Procédé de séquençage d'ADN selon la méthode à plusieurs amorces, caractérisé en ce que :
(a) dans l'ADN à séquencer, on sélectionne un site de fixation d'amorce, qui est un site simple brin de séquence connue,
(b) d'une manière directement adjacente à ce site de fixation d'amorce, on procède à l'hybridation de deux oligonucléotides courts, lesquels sont des oligonucléotides hexamériques, heptamériques, octamériques ou nonamériques,
(c) éventuellement en même temps que l'opération (b) ou après l'opération (b), on procède à l'hybridation, sur le site de fixation d'amorce, d'un autre oligonucléotide hexamérique, heptamérique, octamérique ou nonamérique d'une manière directement adjacente à l'un des deux autres oligonucléotides et
(d) on procède à la ligation des oligonucléotides de façon à former une amorce.

2. Procédé selon la revendication 1, caractérisé en ce qu'on procède à l'hybridation de deux oligonucléotides sur le site de fixation d'amorce et on procède à leur ligation de façon à former une pré-amorce et, seulement après, on procède à l'hybridation d'un troisième oligomère sur le site de fixation d'amorce et à la ligation avec la pré-amorce.

3. Procédé selon l'une des revendications 1 et 2, caractérisé en ce que, pour la formation de l'amorce, on utilise des oligonucléotides hexamériques, heptamériques, octamériques et/ou nonamériques.

4. Procédé selon l'une des revendications précédentes. caractérisé en ce qu'on utilise des oligonucléotides phosphorylés à leur extrémité 5', lorsque cette extrémité doit faire l'objet d'une ligation avec un oligonucléotide voisin au moyen d'ADN ligase T4.

5. Procédé selon l'une des revendications précédentes. caractérisé en ce qu'après la ligation et avant le séquençage. on élimine, par traitement thermique, des oligonucléotides qui n'ont pas fait l'objet d'une ligation donnant l'amorce voulue.

6. Banque d'oligonucléotides, pour la mise en oeuvre d'un procédé selon l'une des revendications précédentes, comprenant la totalité des 4⁶ oligonucléotides hexamériques différents possibles.

7. Banque d'oligonucléotides, pour la mise en oeuvre d'un procédé selon l'une des revendications précédentes, comprenant la totalité des 4⁷ oligonucléotides heptamériques différents possibles.

8. Banque d'oligonucléotides, pour la mise en oeuvre d'un procédé selon l'une des revendications précédentes, comprenant la totalité des 4⁸ oligonucléotides octamériques différents possibles.

9. Banque d'oligonucléotides, pour la mise en oeuvre d'un procédé selon l'une des revendications précédentes, comprenant la totalité des 4⁹ oligonucléotides nonamériques différents possibles.
